# EUROPEAN PATENT SPECIFICATION

(11) **EP 3 386 433 B1**
(45) Date of publication and mention of the grant of the patent: **13.11.2024**
(21) Application number: 15910402.5
(22) Date of filing: 10.12.2015
(51) Int. Cl.: A61F 2/01

(54) **IVC FILTER RETRIEVAL SYSTEMS WITH MULTIPLE CAPTURE MODES**
IVC-FILTERRÜCKGEWINNUNGSSYSTEME MIT MEHREREN ERFASSUNGSMODI
SYSTÈMES D'EXTRACTION DE FILTRES VCI À MULTIPLES MODES DE CAPTURE

(43) Date of publication of application: 17.10.2018
(73) Proprietor: Avantec Vascular Corporation, Sunnyvale, CA 94085 (US)
(72) Inventor: RUVALCABA, Teresa, Sunnyvale California 94085 (US); BECKING, Frank, Los Gatos California 95031 (US); HALDEN, Karl, Sunnyvale California 94085 (US)
(74) Representative: Mathys & Squire
(86) International application number: PCT/US2015/065102
(87) International publication number: WO 2017/099786

(56) References cited:
- US-A- 5 190 561
- US-A- 5 370 647
- US-A1- 2004 181 237
- US-A1- 2005 159 770
- US-A1- 2011 040 321
- US-A1- 2012 010 699
- US-A1- 2015 105 819
- US-A1- 2015 105 819
- US-B2- 6 602 271
- US-B2- 7 993 362
- US-B2- 8 475 488

## Description

### FIELD

The embodiments described herein relate to endovascular temporary Inferior Vena Cava (IVC) filter, other implant retrieval devices or system

### BACKGROUND

Temporary IVC filters are placed much like permanent filters, but are designed so that they may be retrieved in a separate endovascular procedure, generally from a femoral vein or internal jugular vein approach. Most of the currently available temporary filters include a hook-like feature with which they can be captured and received within a catheter or sheath for removal by employing a gooseneck snare or a multi-loop snare.

While retrieval is a simple procedure in principle, difficulty is often encountered capturing a filter's hook with the snare loop(s). Such difficulty is compounded when the filter is tilted or off-kilter in placement. Several filters are designed to avoid such orientation. However, the problem remains common because the device is not anchored into the IVC in a stable fashion. Constant blood flow in addition to blood clots can disorient the filter within the IVC making recapture difficult. Accordingly, there exists a need for a filter retrieval system with improved ease of use and/or less susceptibility to problems of filter orientation. US2015105819 discloses devices including braid for delivery and/or retrieval of filters or other medical devices. US2012010699 discloses a method and apparatus for facilitating transapical removal of a prosthetic heart valve. US5370647 discloses a tissue and organ extractor. US2004181237 discloses a medical device for manipulation of a medical implant. US 5,190,561 discloses a device for extraction of tissue and organs during laparoscopic procedures.

### SUMMARY

The invention is defined in the appended independent claim with optional features set out in the dependent claims. Embodiments hereof meet this need and others as applied to other medical device applications. For IVC filters, the subject systems may be used with a wide variety of filter architectures -- existing or otherwise. Accordingly, new filters may be designed for use with the subject retrievers in which fewer design constraints and/or compromises may be required of the filter design. Features of the subject system may be used in connection with existing and/or modified versions of the filters described in any of USPNs 3,952,747; 5,601,595; 6,443,972; 7,338,512 and 7,625,390, with commercially available devices including the OPTEASE, GUNTHER TULIP, CELECT and OPTION or others.

Embodiments hereof share a "funnel-trap" type architecture. This is advantageously constructed of heatset braid, possibly superelastic (SE) nickel-titanium alloy (Nitinol) braid. The funnel-trap end of a retrieval device includes a distal rim defining a distal opening, and a more proximal aperture or opening. A pocket is formed between the proximal opening and sides of the braid (or other material from which the device is constructed).

When an enlarged proximal end of an IVC filter or other implant is guided past the distal rim of the funnel shape it passes through the proximal opening for capture. Such an enlargement by be in the form of a nub bin or bump or a hook-type interface. In one example a locking sheath is advanced over and closes the trap to secure the enlarged end of the medical device in a pocket adjacent to the proximal opening or aperture. In another embodiment, the proximal aperture is cinched closed to effect capture. In yet another embodiment, crossing members act like a web to catch or entangle any hook or other feature passing into or through the proximal opening aperture.

The subject delivery and/or retrieval devices, kits in which they are included (with and without assembly), methods of use and manufacture (including assembly of the constituent components *in viva* or ex *viva)* are included for information only in the present disclosure. Some aspects of the same are described above, and more detailed discussion is presented in connection with the figures below.

Other systems, devices, methods, features and advantages of the subject matter described herein will be or will become apparent to one with skill in the art upon examination of the following figures and detailed description.

### BRIEF DESCRIPTION OF THE DRAWINGS

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
Figs. 1A and 1B picture IVC filter variations as may be used in the present system.
Fig. 2 is a side view of a delivery and/or retrieval system with an end of any type of implantable medical device or foreign body.
Fig. 3 is a side-sectional view of a converted preform (i.e., a finally shaped funnel section of the subject device) after heatsetting.
Fig. 4 is a partial side-sectional view of retrieval system embodiment including a loop and tether.
Fig. 5 is a side view of a partially inverted preform and associated tether or loop.
Fig. 6 is a side view of a portion of a tether and setup for splicing.
Figs. 7A-7D are top or end view illustrations of various loop and tether construction options.
Fig. 8 is an end view of a funnel trap with crossing member and tether installed.
Figs. 9A-9C are top or end view illustrations of various crossing member and tether construction options.
Fig. 10 is a side sectional view of a retrieval system embodiment including crossing member(s) and an optional tether.
Fig. 11 is a flow diagram depicting an example of a method of use, which does not form part of the claimed subject matter.

### DETAILED DESCRIPTION

Various exemplary embodiments are described below. Reference is made to these examples in a non-limiting sense, as it should be noted that they are provided to illustrate more broadly applicable aspects of the apparatuses and systems. Various changes may be made to these embodiments and equivalents may be substituted without departing from the scope of the invention as defined in the appended claims .

Before the present subject matter is described in detail, it is to be understood that this disclosure is not limited to the particular example embodiments described, as such may, of course, vary. It is also to be understood that the terminology used herein is for the purpose of describing particular embodiments only, and is not intended to be limiting, since the scope of the present disclosure will be limited only by the appended claims.

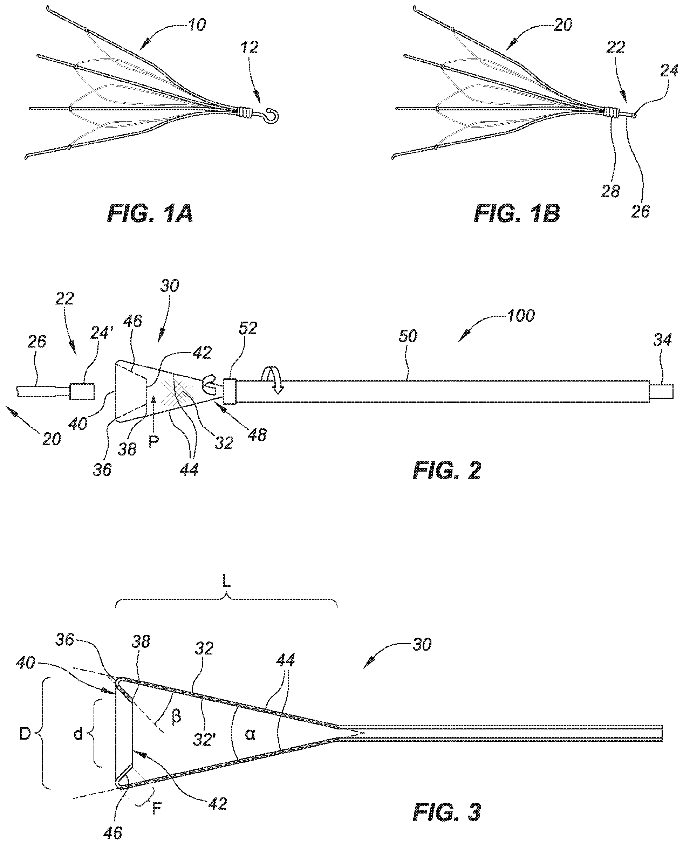

Fig. 1A shows a GUNTHER TULIP (Cook Medical, Inc.) temporary IVC filter 10 with a hook 12 end interface for retrieval. As shown in Fig. 1B for a IVC filter 20, the hook may be modified or substituted for a nubbin-type interface 22. The nubbin (itself) may comprise a laser-formed or solder-formed protuberance or bump 24 on an extension 26 from a hub 28. Alternatively, as shown in Fig. 2, a/the filter retrieval interface 22 may comprise a band 24' (e.g., a Pt marker band) mounted (e.g., by swaging, welding, gluing, etc.) on a/the extension 26. However the enlargement is created, the funnel-trap structures described below are adapted to secure that feature for IVC filter retrieval.

Fig. 2 provides an overview of the subject system 100. A funnel-trap structure 30 is shown made of heatset braid material 32. The construction provides a flexible distal extension to an elongate shaft 34. The shaft is received within an elongate sleeve 50 (that may be a commercially available catheter or sheath or a custom part of the overall system 100) and may include a distal radiopaque marker band 52.

The braid may comprise Nitinol (preferably that is superelastic (SE) at human body temperature), CoCr, Stainless Steel or another biocompatible material. It is advantageously braided material incorporating between 72 and 288, or between about 144 and 192 filament "ends" in a 1-over-1, 1-over-2, 2-over-2 or other pattern. With (superelastic) Nitinol, the wire is advantageously between about 0.001 and about 0.003 inches in diameter. In which case, a supple and relatively "smooth" matrix surface is provided from which to construct the flexible funnel-trap architecture shown and described. The value of such a surface is in its atraumatic aspect and/or ability to help guide an IVC filter interface into position for capture even if it is oriented off-angle. Still, other wire sizes and/or end counts in a braid or other construction options are possible as well.

To assist with target device capture or recapture, the funnel trap structure 30 may be selectably directable. As indicated by the arrows in Fig. 2, the material from which it is made can be heatset or otherwise configured to provide a bias in an angular direction. The angle of deployment may be selectable or fully straightened by relative position of a core member or obturator (not shown) or by a sleeve or catheter sheath as further described. Further positioning may be achieved by rotating the device as further illustrated. Alternatively, a curved, "L" or "J" shaped wire may be received within a lumen of shaft 34 that can be passed up to and/or through to the inside of the funnel trap structure. Made of superelastic Nitinol (or other) wire, this member can be used to selectively shape or direct the device end. Likewise, a shaped catheter or sheath can be employed for such purposes.

Other device articulation options for selecting the angular orientation of the funnel-trap portion of the device are possible as well. Any of a variety of steerable or directable catheter-type technologies (reliant on pull-wires or otherwise) can be incorporated in shaft 34 for such purposes. Examples include the mechanisms described in USPNs 4,723,936; 4,960,411; 6,251,092 and 8,273,073.

The "funnel trap" may be generally frusto-conical in shape as shown or otherwise configured. With an outer conical shape (i.e., a triangular shape in cross section as shown in Fig. 3) the structure is highly supportive for any necessary or desirable tissue discretion that might need to occur to free an emplaced filter. Moreover, such a shape provides a flexible "waist" section 48 for the directable feature(s) noted above. Still, the device may be bowed outward along its sides or otherwise configured without departing from claimed inventive aspects of variations.

Importantly, the distal rim opening 40 of the structure is larger than the more proximal rim opening or aperture 42 to operate in guiding filter engagement feature(s) or enlargement 24/24' (as shown) past the proximal opening or aperture into a pocket (P) where it is captured and subsequently locked upon advancing sleeve 50.

Such a pocket is formed between braid walls 44 and bend or fold 38 in the braid, for which the fold optionally serves as an abutment feature with an edge or shoulder of nub bin/bump 24/24' when the funnel trap section 30 is compressed or collapsed. To ensure capture, the sleeve 50 may be advanced fully over trap 30 before withdrawal into a separate catheter. In other words, in some embodiments, advancing sleeve 50 over funnel section 30 "closes the trap" and securely captures the implant to be retrieved.

Sleeve 50 may be a dedicated part of system 100 or it may be a catheter or so-called jugular access sheath. After the medical device (as in the illustrated case a temporary IVC filter) is covered by advancing the sleeve 50 over it, then - typically - the medical device is retrieved by withdrawal into this sleeve, catheter or sheath 50 or another catheter (not shown). Any or all such activity may be visualized fluoroscopically by a physician by way of marker features 24/24' and 52 and/or others as may be conveniently provided.

Notably, system 100 may be used identically when capturing a filter 10 with a typical hook end 12. However, the additional bulk/lateral extension of the hook may necessitate use of a relatively larger sleeve or catheter 50 for locking.

In the various system architectures, the catheter/pusher shaft 34, sleeve 50 or other catheters or sheaths used in or with the system may comprise a simple extrusion (e.g., PTFE, FEP, PEEK, PI, etc.) or may be constructed using conventional catheter construction techniques and include a liner, braid support and outer jacket (not shown), metal hypotube, etc. Further, the filter frame may be constructed using conventional laser cutting and electropolishing techniques and/or be otherwise constructed. In embodiments intended for tracking through a guide/delivery catheter without an incorporated sheath, a loading sheath may be employed. Any such loading sheath may be splittable. Other typical percutaneous access instruments (such as wires, etc.), valves and other hardware may also be employed in connection with the invention embodiments, including medical treatment methods.

The funnel-trap structure 30 can be made as a subassembly and attached to its catheter/pusher shaft 34. PCT publication PCT/US2014/042343 (WO2014201380) and U.S. Patent Application No. 14/569,500, each detail optional steps in the manufacture of a braid preform of the funnel-trap portion 30 of the final device as shown in Fig. 3.

Here, inner and outer layers of braid 32 are shown heatset using conventional techniques (e.g., in a furnace, salt pot, etc.) in a funnel shape with distal bends 36 in the braid wire forming an outer rim 40 with a large(r) distal opening or aperture. Inner or proximal bends 38 form an inner rim 42 with a small( er) more proximal opening or aperture. Stated otherwise, the braid used to construct the funnel-shape trap is folded back (e.g., in a flap 46) at the distal opening to provide a more proximal opening or aperture. Likewise, the braid is folded over or back to define the proximal opening or aperture.

For IVC filter retrieval, the funnel-trap portion 30 shown may have a diameter (D) from about 5 mm to about 20 mm, or more preferably about 10 to about 15 mm (i.e., size in a range to work within average size human IVCs where such vessels are reported as having a mean diameter of 20 mm within a range of 13 to 30 mm). A length (L) may range from about 10 mm to about 30 mm. An overall cone angle (a) between braid walls 44 may be between about 30 and about 90 degrees. An angle *W)* of bend 36 between braid wall 44 and flap 46 may be between about 0 and about 60 degrees and flap length (F) may be between about 1 and about 10 mm in length. Overall, an opening diameter (d) may be between about 5 and about 95 percent of diameter (D) depending on the selected combination of the noted variables (i.e., d, D, L, F, α and β).At the lower end of this range, the inner "opening" may be substantially closed such that it must be pushed-open to receive the proximal engagement feature(s) of the implant during retrieval. At the higher end of the range, the flap may lie completely along or in-line with the outer layer(s) of the device. The configuration selected will depend upon the type of capture approach selected as further detailed herein. The opening 40 of the funnel trap may be set perpendicular relative to a device axis (A) as shown. Otherwise, it may be angled or have a more complex shape as described in connection with the above-referenced US Patent Application 14/569,500.

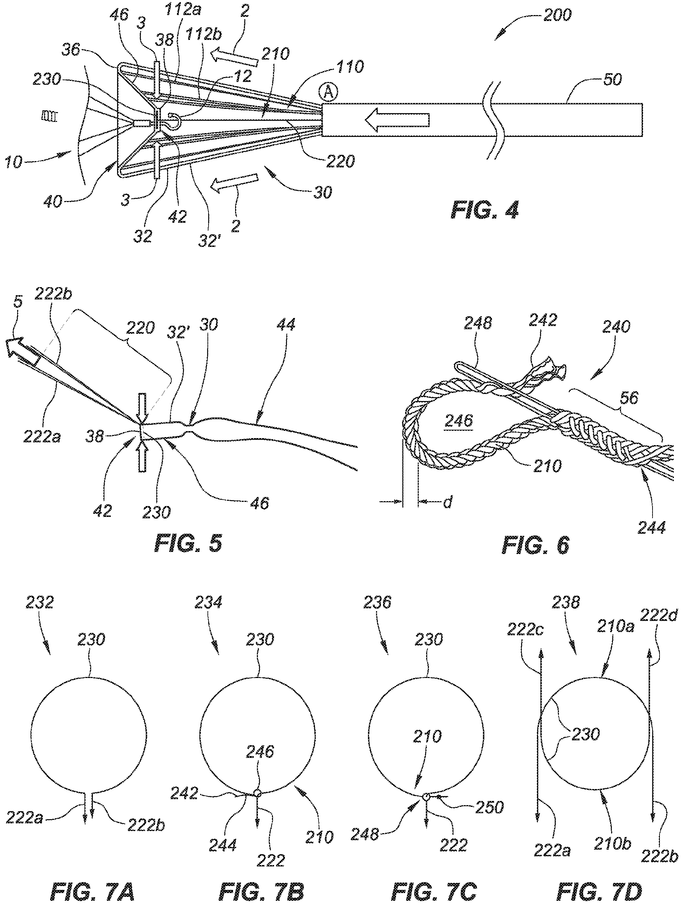

As shown in Fig. 4, embodiments hereof may include a support member 110 including elongate members 112a, 112b, etc. set within the funnel trap section or portion of the device to support distal rim 40. This example shown in cross section may have eight such elongate members connected with four each to a nested or stacked hub portion (not shown). The support member(s) may alternatively or additionally be interposed between braid layers 32, 32'. Further details of possible support member constructions and/or placements are set forth in PCT Patent Application No. PCT/US 15/65025 and U.S. Patent Application No. 14/965,500, both of which are titled 'TVC Filter Retrieval Systems with Interposed Support Members,".

In Fig. 4, the system 200 shown includes components as discussed above along with the addition of a line, fiber, filament, fibril, thread, yam or strand 210 serving as a tether 220. In one example, the strand element 210 (as a yarn or thread) is provided as a braided or plaited suture material. Ultra-high-molecular-weight polyethylene (UHMWPE) suture material may be selected for such purpose based on its strength, limited stretch and biocompatible characteristics. Other polymer fiber or metal filament (e.g., Nitinol) options are possible as well.

In any case, the tether can serve any number of purposes. In one example, the tether may hold or stabilize the inner flap 46 from pulling out or everting if the medical device 10 to be captured is one that includes a hook interface 12. A hook 12 can be captured by the funnel trap structure upon passing into or though the proximal rim opening 42. The hook may catch on a crossing filament as discussed further below or along the rim. For such purpose, the rim is supported or supplemented with a ring 230 interposed between braid layers 32/32'. The ring may be defined by a portion of the strand 210 as further described below or otherwise.

In another example, the tether 220 may be used for actuation of the rim opening or aperture 42. When the ring 230 is in the form of a synchable loop or lasso as part of the strand, the tether portion 220 of the strand can be used to close the associated aperture 42. (Examples of such loop or lasso constructions are discussed further below).

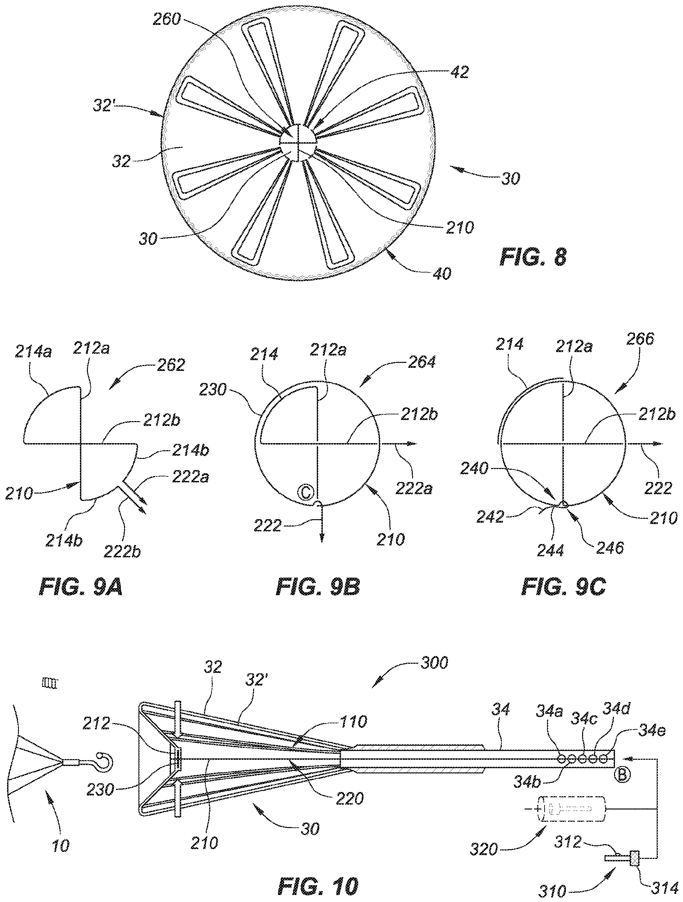

The tether may be actuated (e.g., pulled) using a handle interface (e.g., as shown in Fig. 10). Alternatively, the tether may be affixed anywhere proximal to the funnel trap section 30 and still be used to close a lasso interface for medical device capture. Even if attached at or adjacent to point (A) in Fig. 4, when sheath 50 is advanced as shown over the funnel trap section 30, the braid defining the funnel trap collapses or compresses with the angle of its included wires changing. This causes the funnel section to lengthen. With the tether position fixed, it effectively "pulls" on any lasso member included at the proximal aperture closing it. All such action is indicated by arrow sets 1, 2, 3 in Fig. 4.

The so-called lasso may be included at an intermediate stage of production of the system 200. Fig. 5 shows a loop or lasso 232 configuration as further described in connection with Figs. 7 A. While the loop 230 strand may be threaded into place at the wire bend or fold 38, it is more efficiently installed by pulling apart or folding back braid layer 32 and 32' and inserting it there between. Then, the braid configuration shown is flipped back so that flap section 46 is once again inset within the (optionally) conical shape of the funnel.

As shown in Fig. 7A, a loop pattern 232 is formed that includes a ring 230 and two exit legs or lengths 222a, 222b. These lengths may extend substantially as indicated by the arrows and used as tether members, tied-off to one another and trimmed, one may be tied-off to another and trimmed with the other used at a longer length, etc.

In Fig. 5, loop pattern 232 is set in place between braid layers 32, 32' with its two ends 222a, 222b exiting the same space or nearby spaces in the braid as shown. Pulled as a tether 220, loop 230 inside aperture 42 is synched or closed down (as indicated by arrows 5 and 3, respectively).

Another approach in defining a synchable loop is to use a slipknot or eyelet in the strand so that a single entry/exit member can be used passing through the braid. Fig. 6 is a side view of a portion of a suture or other fiber strand 210 setup for splicing-in such an eyelet. Sometimes the type of splice 240 to be constructed with the setup shown is referred to as a "long buried" splice. With hollow braided (in this case) suture material, an elongate tail 242 section of the material can be drawn through a splice channel 244 opened in the strand of material to define an eye or eyelet region 246. A needle or wire tool 248 may be used to draw the tail through the body of the strand 210.

With a ratio of splice length (SL) to strand diameter ( d) of about 3 5 to 70 times or greater, (i.e., SL in the case of the suture material described below of about 5 to 6 mm long) splice strength on par with the native material can be achieved. For added security, the splice channel or section 244 can be further stabilized by biocompatible glue, laser welding or heat staking. Still further, any remaining tail 242 length of the splice can be melt-formed into a ball (not shown) that will not pull through the braided body. Other variations in the splicing procedure may include removing a number of filaments from the tail section to reduce the splice bulk. However, when dealing with fine suture (e.g., on the order of about 0.008 to about 0.012 inches (or about 0.2 to about 0.3 mm) in diameter with as few as 6 or 8 braided threads as advantageously used in embodiments hereof) such activity may be avoided.

Referring specifically to Fig. 7B, it illustrates loop pattern 234 constructed with such a splicing approach. A single strand 210 passes through the eyelet 246 formed by splicing. The end length 222 may serve for a tether or other features as further described.

Fig. 7C shows a loop pattern or approach 236 where an eyelet 248 is formed by splitting or passing the strand 210 through itself The end of the strand opposite any retained end length 222 may be tied-off by a knot, weld or glue bead 250.

Fig. 7D presents yet another loop or lasso pattern 236. Here, two strands 210a and 210b are provided to encircle a funnel aperture 42. This approach may offer improved syncing around the fold 38 in which it may be placed. However, with as many as four tether ends 222a-222d to manage for assembly, other options may be preferred.

Fig. 8 is an end view of a funnel trap 30 variation that may include a ring 230, optionally in the form of a synchable loop or lasso. Such a construction may be placed between braid layers 32/32'. Or at least one strand 210 may be used to define a cross-hairs type structure 260. More generally, one, two or more aperture crossing strands may be installed or threaded through or with the braid to serve as a filter hook 12 capture interface.

With multiple crossing strands or members, a "web" for implant capture may be defined. The web may use regularly (i.e., consistently) spaced members. Or it may be asymmetrical. The web may include two segments crossing in an "X" pattern (e.g., as shown in Fig. 8). Another example may have three segments defining a "Y" shape. Yet another example pattern is in the shape of a trefoil knot. Still others options are possible as well.

Particular tying or threading patterns for X- or crossing-type webs are presented in Figs. 9A-9C. A simple cross or X type interface (i.e., without a surrounding loop or ring) may be constructed with a pattern 262 as show in Fig. 9A. As sort of a figure-eight threading pattern through the braid (with crossing sections 212a and 212b and curve or turn sections 214a, 214b) set between or outside braid layers 32, 32' includes two end lengths (222a, 222b ). These may be tied off to one another in a knot (not shown), they may pass proximally to a tie-off or glue-in point, or be otherwise managed or handled.

Fig. 10 illustrates an example of a proximal end glue-in attachment point or zone (B) where shaft 34 includes ports 34a-34f. These ports may receive adhesive for an improved physical lock with shaft body and/or simply provide visual indication of formation of an adequate length glue joint to hold tether member(s) securely within the shaft.

Fig. 9B shows an alternative tie pattern for a loop and crosshair arrangement 264. The loop or ring 230 defined (when placed between braid layers) supports any filter (or other implant) hook received in or around the rim of the funnel trap device. With such a ring in place, a hook cannot simply pull through a number of captured or entrained braid filaments (a consideration when using fine Nitinol or other filaments in the braid). Rather, the hook would have to pull past the strand 210 itself (which is also supported by braid all around). Regardless, the hook may sometimes instead catch or locate upon the crossing members 212a, 212b of the pattern. They are supported from pull-out by the ends 222a, 222b that may be secured to one another by tying or otherwise as per variations discussed above.

As for other details of pattern 264, note the looping-around or intertwining of strand sections indicated at (C) that may assist in providing a more stable loop structure. Also, note the center region in which the horizontal member 212b (as oriented in the view) appears to cross over the vertical member 212a, and the strand portion adjacent extensions 222a under adjacent loop 230 section. Such up/down or over/under weaving may advantageously be employed to control strand portion position or placement within the finally constructed device.

Similar or related weaving is shown for pattern 266 shown in Fig. 9C. What differs primarily here is the use of a strand 210 with a splice 240 and splice-defined eyelet 246 so that a structure similar to that in Fig. 9B can be produced with a single end 222 where this end may ultimately serve as a tether or be tied-off at the aperture (to the strand, adjacent braid or otherwise).

Referring again Fig. 10, it provides a side sectional view of a retrieval system embodiment 300 including crossing member(s) 212, a ring 230 around proximal opening rim 42, and an optional tether(s) 220. The tether glue-in approach illustrated has already been discussed. Also noteworthy is the manner in which the setup is configured to release the tether if disengagement of a/the filter is desired. Simply by cutting shaft 34, the tether 210 is released and can be pulled free of filter hook 12 engagement. In another arrangement, a plug 310 may be used to secure or hold the tether in place in system 300 (or system 200) until removed. The plug may be press fit (lightly so as to allow removal), threaded or held with a detent 312 in place. A textured or knurled grip 314 may be provided for user-interface purposes. Other options are possible as well.

But once the plug is pulled or the shaft is cut, tether 210 is released. Especially when a spliced-eyelet tether is used with its loop or ring set between braid layers, such release does not risk losing the tether strand 210 altogether from system 200 and/or 300.

Also notable is that system 300 can advantageously be used to capture a filter 10 without a locking catheter (note that none is shown in Fig. 10). Still, cover and withdrawal of any captured IVC filter will commonly be accomplished in connection with a commercially available catheter or sheath.

Still further, an optional handle 320 (indicated by dashed line at small scale) may be included in the system for any desired tether manipulation. With such a handle, an opening interface selected from any of the patterns presented in Figs. 7A-7D, 9A-9C or related may be closed by pulling the tether through handle manipulation (e.g., via a wheel, thumb slide or other user interface feature). Also, the handle may be configured to release tether 210 like plug 310 described above and/or let-out some portion of tether length (e.g., up to about 0.5 inches or about 10 mm to about 15 mm or more) to allow flap 46 and aperture 42 eversion for any efforts that may be desired to release a hook 12 from the system.

### Methods

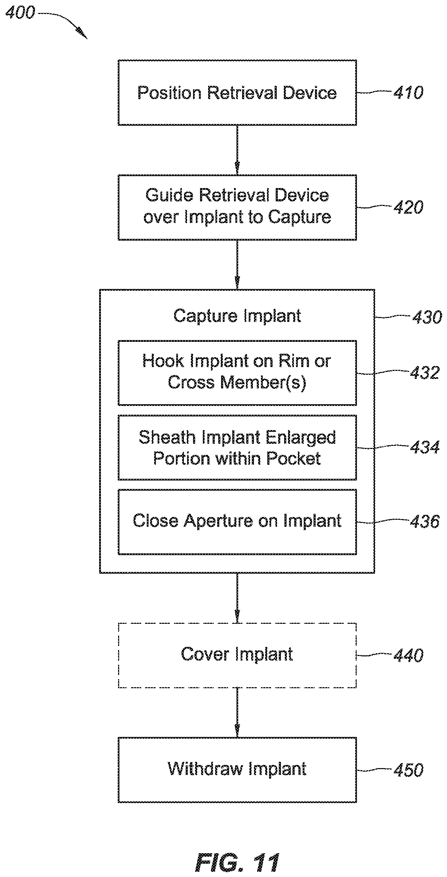

Various methods 400 of use can be defined in connection with the subject funnel trap embodiments 100, 200 or 300 or related medical device hardware. All such methods are not part of the claimed subject matter, but are discussed to aid understanding of the invention. Fig. 11 is a flow diagram depicting one example embodiment of a method of use. Vascular access, medical imaging and positioning of the selected device adjacent to an implant or foreign body to be retrieved in the patient's vasculature are steps common to the methods. For example, see 410 in Fig. 11. Such positioning 410 is achieved by or after passing the funnel trap device through a sleeve in the form of a vascular access sheath or catheter.

Likewise, at 420, the methods include positioning the funnel trap device over the proximal portion of the element to be captured. In doing so (i.e., for the case of filter retrieval), the proximal capture interface of the filter will pass first through a distal aperture and then through the proximal aperture of the funnel-trap device. Due to the braided construction offered, such passage may be regarded both as protected (i.e., as the implant capture feature of the implant is within the boundary defined by braid) and self-guided (i.e., as the capture feature moves past or along the inner flap section of the funnel trap).

At 430, a number of capture options are possible depending on the system selected for use. Per option 432, the proximal interface may be a hook captured by a crossing member or along the rim interface at the proximal aperture of the funnel trap retrieval device. As another option 434, a loop or lasso feature at the proximal aperture may be synched or tightened around the implant capture feature (be it a hook or other enlarged portion of an IVC filter). As yet another option 436 (i.e., in connection with advancing a locking sheath or catheter), the implant capture feature may be secured within a pocket of the device at or adjacent to the aperture and its nm.

After any such action, a/the catheter is typically advanced to cover the IVC filter at 440. During such advancement, tissue may separate from the body or legs of the filter (i.e., if this is the type of implant being capture, other possibilities include lost or stray embolization coils, part of a Central Venous Catheter (CVC) or line, etc ). Finally, at 450, the filter (or other medical device) is withdrawn through a catheter.

Both the advancement to cover the implant (or other device to be retrieved or recovered) and withdrawal may take place in connection with one catheter. However, when a separate locking catheter is provided (e.g., in connection with system 100 for the approach in 434) an inner locking catheter will typically be what is advanced over the implant or device to be retrieved, and it will be withdrawn - together with the implant or device retrieved - out through an outer access catheter or sheath (i.e., the catheter or sheath originally used to achieve vascular access).

Clearly, a single-catheter approach can save procedure and/or fluoroscopy time. Systems 200 and 300 may be better suited to realize such advantages in view of their additional implant or device (e.g., IVC filter) capture features they possess as variously described above. However, each of the system embodiments described herein presents its own unique advantages that argue for its use and clinical relevance and/or adoption, especially in comparison to known retrieval devices and approaches.

### Variations

The subject methods, including methods of use and/or manufacture are not part of the claimed subject matter, and may be carried out in any order of the events which is logically possible, as well as any recited order of events.

Methods may include any of a hospital staffs activities associated with device provision, implant positioning, re-positioning, implant or device retrieval and/or release.

Furthermore, where a range of values is provided, it is understood that every intervening value, between the upper and lower limit of that range and any other stated or intervening value in the stated range is encompassed within the invention. Also, it is contemplated that any optional feature of the inventive variations described may be set forth and claimed independently, or in combination with any one or more of the features described herein.

Though the invention has been described in reference to several examples, optionally incorporating various features, the invention is not to be limited to that which is described or indicated as contemplated with respect to each variation of the invention. Various changes may be made to the invention described and equivalents (whether recited herein or not included for the sake of some brevity) may be substituted without departing from the true scope of the invention as set out in the appended claims.

## Claims

1. Retrieval apparatus (100, 200, 300) for a vascular medical device, the apparatus comprising:
an elongate shaft (34) having an axis having a flexible distal extension comprising braid (32), the distal extension folded-back inwardly to form a distal opening (40) and a proximal opening (42), the proximal opening sized to receive a portion of the medical device therethrough for securing the medical device, **characterised in that** the apparatus further comprises:
at least one a ring member (230) positioned between two layers of the braid (32, 32') encircling the proximal opening.

2. The apparatus (100, 200, 300) of claim 1, wherein the ring member (230) comprises at least one flexible strand of material (210).

3. The apparatus (100, 200, 300) of claim 2, wherein the at least one strand (210) is configured to at least partially close the proximal opening (42) upon actuation, optionally wherein the at least one strand is a single strand including an eyelet splice (246) at a distal end.

4. The apparatus (100, 200, 300) of claim 2, wherein a tether portion or tether portions of the at least one strand (210) is connected to the apparatus proximal to the proximal opening (42), optionally wherein the tether portion or tether portions are connected at a proximal end of the elongate shaft (34), optionally wherein the braid (32) of the extension section is configured so advancement of the elongate member closes the proximal opening by elongation of the distal extension relative to the tether portion or tether portions, optionally further comprising a user interface at a proximal end of the shaft and connected to the tether portion or tether portions to actuate the tether portion or tether portions for closing the opening or release, the user interface selected from a handle and a plug received by the elongate shaft.

5. The apparatus (100, 200, 300) of claim 1, further comprising at least one member (212a, 212b) positioned to cross the ring member, optionally wherein two members cross the ring member in an X pattern.

6. The apparatus (100, 200, 300) of claim 2 or 4, wherein the at least one flexible strand (210) crosses the proximal opening, optionally wherein the at least one flexible strand crosses the proximal opening in an X pattern.

7. The apparatus (100, 200, 300) of claim 1, further comprising at least one flexible strand (210) positioned to cross the proximal opening, optionally wherein the at least one strand crosses the opening in an X pattern.

8. The apparatus (100, 200, 300) of claim 7, wherein a tether portion or tether portions of the at least one strand (210) is connected to the apparatus proximal to the proximal opening (42), optionally wherein the tether portion or tether portions are connected at a proximal end of the elongate shaft (34), optionally wherein the flexible member is also positioned between two layers of the braid (32, 32') to encircle the proximal opening, optionally wherein the at least one strand is a single strand including an eyelet splice (246) at a distal end.

## Patentansprüche

1. Wiederentnahmevorrichtung (100, 200, 300) für ein vaskuläres medizinisches Gerät, wobei die Vorrichtung Folgendes umfasst:
einen länglichen Schaft (34) mit einer Achse mit einer flexiblen distalen Erweiterung, die ein Geflecht (32) umfasst, wobei die distale Erweiterung nach innen zurückgefaltet ist, um eine distale Öffnung (40) und eine proximale Öffnung (42) zu bilden, wobei die proximale Öffnung dazu bemessen ist, einen Anteil des medizinischen Geräts durch sie hindurch aufzunehmen, um das medizinische Gerät zu sichern, **dadurch gekennzeichnet, dass** die Vorrichtung ferner Folgendes umfasst:
mindestens ein Ringelement (230), das zwischen zwei Schichten des Geflechts (32, 32') positioniert ist, das die proximale Öffnung kreisförmig umgibt.

2. Vorrichtung (100, 200, 300) nach Anspruch 1, wobei das Ringelement (230) mindestens einen flexiblen Materialstrang (210) umfasst.

3. Vorrichtung (100, 200, 300) nach Anspruch 2, wobei der mindestens eine Strang (210) dazu ausgelegt ist, die proximale Öffnung (42) nach der Betätigung mindestens teilweise zu verschließen, wobei der mindestens eine Strang optional ein einzelner Strang ist, der an einem distalen Ende einen Ösenspleiß (246) einschließt.

4. Vorrichtung (100, 200, 300) nach Anspruch 2, wobei ein Anbindungsanteil oder Anbindungsanteile des mindestens einen Strangs (210) proximal der proximalen Öffnung (42) mit der Vorrichtung verbunden sind, wobei der Anbindungsanteil oder die Anbindungsanteile optional an einem proximalen Ende des länglichen Schafts (34) verbunden sind, wobei das Geflecht (32) des Erweiterungsabschnitts optional so ausgelegt ist, dass das Vorschieben des länglichen Elements die proximale Öffnung durch Verlängern der distalen Erweiterung relativ zu dem Anbindungsanteil oder den Anbindungsanteilen verschließt, die ferner optional eine an einem proximalen Ende des Schafts liegende und mit dem Anbindungsanteil oder den Anbindungsanteilen verbundene Benutzerschnittstelle umfasst, um den Anbindungsanteil oder die Anbindungsanteile zum Schließen der Öffnung zu betätigen oder freizugeben, wobei die Benutzerschnittstelle aus einem Handgriff und einem Stecker, der von dem länglichen Schaft aufgenommen wird, ausgewählt ist.

5. Vorrichtung (100, 200, 300) nach Anspruch 1, die ferner mindestens ein zum Queren des Ringelements positioniertes Element (212a, 212b) umfasst, wobei optional zwei Elemente das Ringelement in einem x-förmigen Muster queren.

6. Vorrichtung (100, 200, 300) nach Anspruch 2 oder 4, wobei der mindestens eine flexible Strang (210) die proximale Öffnung quert, wobei optional der mindestens eine flexible Strang die proximale Öffnung in einem x-förmigen Muster quert.

7. Vorrichtung (100, 200, 300) nach Anspruch 1, die ferner mindestens einen zum Queren der proximalen Öffnung positionierten flexiblen Strang (210) umfasst, wobei der mindestens eine Strang optional die Öffnung in einem x-förmigen Muster quert.

8. Vorrichtung (100, 200, 300) nach Anspruch 7, wobei ein Anbindungsanteil oder Anbindungsanteile des mindestens einen Strangs (210) proximal der proximalen Öffnung (42) mit der Vorrichtung verbunden sind, wobei der Anbindungsanteil oder die Anbindungsanteile optional an einem proximalen Ende des länglichen Schafts (34) verbunden sind, wobei das flexible Element optional auch zwischen zwei Schichten des Geflechts (32, 32') positioniert ist, um die proximale Öffnung kreisförmig zu umgeben, wobei optional der mindestens eine Strang ein einzelner Strang ist, der an einem distalen Ende einen Ösenspleiß (246) einschließt.

## Revendications

1. Appareil d'extraction (100, 200, 300) pour un dispositif médical vasculaire, l'appareil comprenant:
une tige allongée (34) ayant un axe comportant un prolongement distal flexible comprenant une tresse (32), le prolongement distal étant replié vers l'intérieur pour former une ouverture distale (40) et une ouverture proximale (42), l'ouverture proximale étant dimensionnée pour recevoir une partie du dispositif médical à travers celle-ci pour bloquer en place le dispositif médical, **caractérisé en ce que** l'appareil comprend en outre:
au moins un élément annulaire (230) positionné entre deux couches de la tresse (32, 32') encerclant l'ouverture proximale.

2. Appareil (100, 200, 300) selon la revendication 1, dans lequel l'élément annulaire (230) comprend au moins un brin flexible de matériau (210).

3. Appareil (100, 200, 300) selon la revendication 2, dans lequel l'au moins un brin (210) est configuré pour fermer au moins partiellement l'ouverture proximale (42) lors de l'actionnement, optionnellement dans lequel l'au moins un brin est un brin unique comprenant une épissure d'œillet (246) à une extrémité distale.

4. Appareil (100, 200, 300) selon la revendication 2, dans lequel une partie d'attache ou des parties d'attache de l'au moins un brin (210) sont reliées à l'appareil proximalement à l'ouverture proximale (42), optionnellement dans lequel la partie d'attache ou les parties d'attache sont reliées à une extrémité proximale de la tige allongée (34), optionnellement dans lequel la tresse (32) de la section correspondant au prolongement est configurée de telle sorte que l'avancement de l'élément allongé ferme l'ouverture proximale par allongement du prolongement distal par rapport à la partie d'attache ou aux parties d'attache, comprenant optionnellement en outre une interface utilisateur à une extrémité proximale de la tige et connectée à la partie d'attache ou aux parties d'attache pour actionner la partie d'attache ou les parties d'attache pour produire une fermeture de l'ouverture ou une libération, l'interface utilisateur étant sélectionnée parmi une poignée et un bouchon qui vient se loger dans la tige allongée.

5. Appareil (100, 200, 300) selon la revendication 1, comprenant en outre au moins un élément (212a, 212b) positionné pour traverser l'élément annulaire, optionnellement dans lequel deux éléments traversent l'élément annulaire selon un motif en X.

6. Appareil (100, 200, 300) selon la revendication 2 ou 4, dans lequel l'au moins un brin flexible (210) traverse l'ouverture proximale, optionnellement dans lequel l'au moins un brin flexible traverse l'ouverture proximale selon un motif en X.

7. Appareil (100, 200, 300) selon la revendication 1, comprenant en outre au moins un brin flexible (210) positionné pour traverser l'ouverture proximale, optionnellement dans lequel l'au moins un brin traverse l'ouverture selon un motif en X.

8. Appareil (100, 200, 300) selon la revendication 7, dans lequel une partie d'attache ou des parties d'attache de l'au moins un brin (210) sont reliées à l'appareil proximalement à l'ouverture proximale (42), optionnellement dans lequel la partie d'attache ou les parties d'attache sont reliées à une extrémité proximale de la tige allongée (34), optionnellement dans lequel l'élément flexible est également positionné entre deux couches de la tresse (32, 32') pour encercler l'ouverture proximale, optionnellement dans lequel l'au moins un brin est un brin unique comprenant une épissure d'œillet (246) à une extrémité distale.
